# EUROPEAN PATENT SPECIFICATION

(11) **EP 2 424 605 B1**
(45) Date of publication and mention of the grant of the patent: **05.06.2013**
(21) Application number: 10721369.6
(22) Date of filing: 27.04.2010
(51) Int. Cl.: A61M 16/06, B32B 5/18, B32B 25/16, B32B 27/34, B32B 27/36, B32B 27/40, C08L 67/04

(54) **Wearable medical support for delivering fluids to the nose**
Tragbare medizinische Halterung zur Abgabe von Flüssigkeiten an die Nase
Support médical portable pour la livraison de fluides vers le nez

(30) Priority: 27.04.2009 EP 09158849
(43) Date of publication of application: 07.03.2012
(73) Proprietor: Tomtec N.V., 2950 Kapellen (BE)
(72) Inventor: ANTHONY, Jean-Michel, B-2950 Kapellen (BE)
(74) Representative: Brants, Johan P.E.
(86) International application number: PCT/EP2010/055643
(87) International publication number: WO 2010/125074

(56) References cited:
- WO-A-2004/108199
- US-A- 4 784 123
- US-A- 6 083 442
- US-A1- 2004 079 374
- US-A1- 2006 005 837
- US-A1- 2006 219 246
- US-A1- 2009 267 261

## Description

### FIELD OF THE INVENTION

The present invention is in the field of a wearable support for the delivery of fluids through the nose of a subject. In particular, it concerns a support that can be worn for the treatment of sleep apnea, or for supporting a catheter providing air to the lungs or liquid nutrition to the stomach.

### BACKGROUND TO THE INVENTION

A variety of supports are known in the art that may be worn by a subject and which secure a tube for the delivery of a fluid (*e.g.* a liquid, a gas) through the nose.

The most common supports are respiratory masks, which are triangular in shape to compliment the nose, and seal against the skin of the subject, as described for instance, in US 2006/0096598. A disadvantage of such masks is the weight and the low level of comfort they afford the wearer. Because they seal using a triangular facial component, pressure is applied to the skin in a concentrated region along the triangle edges. They result in unseemly pressure marks to the skin of the wearer after use, which marks are not only attributable to the profile of the mask, but also to the accompanying straps which pass across the face of the wearer. Such masks may be worn only for limited period of time without the risk of adverse reaction. They are unsuitable for wearing at night, as required by sufferers of sleep apnea for long and repeated periods.

An alternative type of support is for a catheter tubing which is employed for the delivery of fluids such as gas or liquid to the nose. Examples include breathing and feeding tubes which are passed down the wind pipe and esophagus respectively. Such tubes are commonly held in placing using an adhesive strip applied to the skin of the face. However, the strips are not strongly adhesive against the friction-resistive tube walls and so are prone to dislodging when the patient moves, thereby risking the possibility that catheter is displaced. Moreover, adhesive strips commonly elicit an allergic reaction in the wearer. Because the strips are worn on the face, the appearance of an allergic rash is highly undesirable. Moreover, allergic rashes are known to be permanent or reoccur for months or years after the event.

US 2006/0005837 discloses a custom fitted mask configured for coupling to an external gas supply system. The mask comprises a first sheet of deformable material that has been deformed against a user's face surrounding the user's nostrils while the first sheet is in a deformable state to conform substantially optimally to the user's unique facial features. The mask further comprises a fitting adapted for coupling to the external gas supply system; and a second sheet of deformable material configured around the fitting. The second sheet is applied against the fitting while the second sheet is in a deformable state such that the fitting is positioned between the first and second sheets, a contact portion of the second sheet conforming substantially optimally to a contact portion of the first sheet and being bonded to the contact portion of the first sheet. US 2004/0079374 discloses a custom fitted mask, comprising a first thin sheet of deformable material that has been formed and fitted to a first portion of a user's face surrounding the user's nostrils. The mask comprises fittings that have been inserted for coupling the mask to an external gas supply system. In these masks, the fitting is permanently fixed to the mask. This is disadvantageous as the mask has to be discarded as soon as the fitting is damaged (after a multiple use and/or a miss-use for instance).

In view of the problems of the art, the present invention aims to provide a new support that avoids the problems of the prior art.

### SUMMARY OF THE INVENTION

The present invention is related to a wearable medical support (100) for delivery of fluids to the nose of a subject comprising:
- a heat moldable nose mask (10), having a longitudinal direction, formed from a sheet of thermoplastic material configured for individual molding across at least part of the cheek bones of the subject, comprising:
- a nose aperture (12) dimensioned to fit the nose of the subject, and
- a fixture (14, 16) for a strap at each opposing longitudinal end of the mask (10),
   and
- a coupling (20, 20a 40, 45) dismountably fixed to the mask (10) for attachment of one or more tubes for delivery of the fluid to the nose.

Another embodiment of the invention is the support (100) as described in claim 15. other aspects of the invention are described in the dependent claims.

### FIGURE LEGENDS

**FIG. 1** depicts a support of the invention disposed with a dismountable or permanently fixed coupling that is a hollow tubular fitting for attachment to a supply of air, whereby the nose mask has a curved profile prior to molding.
**FIG. 2** depicts a support of the invention as shown in **FIG. 1****,** after molding to fit the contours of the region around the nose. The view is of the front of the mask.
**FIG. 3** depicts a support of the invention as shown in **FIG. 1****,** after molding to fit the contours of the region around the nose. The view is of the back (skin contacting or facing) side of the mask.
**FIG. 4** depicts the support of **FIG. 1****,** highlighting a longitudinal (central) axis **A-A'** of the coupling between its proximal and distal ends.
**FIGs. 5A** to **5D** depict different views of a dismountable coupling that is a hollow tubular fitting provided with a proximal flexible flange: **FIG. 5A** is a view with the proximal end facing the viewer, **FIG. 5B** is a view with the distal end facing the viewer, **FIG. 5C** is a cross-sectional view perpendicular to the longitudinal **(A-A')** axis, **FIG. 5D** is a perspective view.
**FIG. 6** is a cross-sectional view of the coupling perpendicular to the longitudinal, mounted in the aperture of the nose mask **10.**
**FIG. 7** depicts a removable coupling of the invention attached to the support. The view is of the back (skin contacting or facing) side of the mask. The dotted line 84 indicates that the flange can extend to the periphery of the mask.
**FIG. 8** depicts an embodiment of the flange part of a coupling embodiment with indicated dimensions.
**FIGs. 9A** to **9E** depict views of a dismountable coupling that is a hollow tubular fitting provided with a proximal flexible flange: **FIG. 9A** is a view with the proximal end facing the viewer, **FIG. 9B1** is a plan view of the coupling provided with a planar flange, **FIG. 9B2** is a plan view of the coupling provided with a curved flange, **FIG. 9C** is a perspective view.
**FIG. 9D** is a perspective view of the coupling mounted on the mask, **FIG. 9E** is a cross-sectional view perpendicular to the longitudinal **(A-A')** axis.
**FIGs. 10A to 10E** depict views of a dismountable coupling that is a hollow tubular fitting provided with a proximal flexible flange: **FIG. 10A** is a view with the proximal end facing the viewer, **FIG. 10B** is a plan view; **FIG. 10C** is a perspective view. **FIG. 10D** is a perspective view of the coupling mounted on the mask; **FIG. 10E** is a cross-sectional view perpendicular to the longitudinal **(A-A')** axis.
**FIGs. 11A to 11C** depict views of a dismountable coupling that is a hollow tubular fitting provided with a proximal flexible flange and a bellowed region of the passageway: **FIG. 11A** is a view with the proximal end facing the viewer; **FIG. 11B** is a plan view; **FIG. 11C** is a perspective view.
**FIG. 12** depicts a nose mask of the invention adapted to slidably receive the dismountable coupling of **FIGs. 9A to 9E****, 10A to 10E** and **FIGs. 11A** to **11C****.**
**FIG. 13** depicts an alternative embodiment of the flange part of a coupling embodiment with indicated dimensions.
**FIG. 14** depicts a support of the invention disposed with a nozzled coupling for attachment to a supply of air, whereby the nose mask has a curved profile prior to molding.
**FIG. 15** depicts a support of the invention as shown in **FIG. 14****,** after molding to fit the contours of the region around the nose. The view is of the front of the mask.
**FIG. 16** depicts a support of the invention as shown in **FIG. 14****,** after molding to fit the contours of the region around the nose. The view is of the back (skin contacting or facing) side of the mask.
**FIG. 17** depicts a support of the invention suitable for coupling to fluid catheter for insertion into the nose, whereby the nose mask has a curved profile prior to molding.
**FIG. 18** depicts a support of the invention as shown in **FIG. 17****,** after molding to fit the contours of the region around the nose. The view is of the front of the mask.
**FIG. 19** depicts a support of the invention as shown in **FIG. 17****,** after molding to fit the contours of the region around the nose. The view is of the back (skin contacting or facing) side of the mask.
**FIG. 20A and 20B** depicts two alternative embodiments of the mask element of a support of the invention with indicated dimensions.
**FIG. 21** depicts a schematic drawing of a cross section through a sheet of thermoplastic material used to form the nose mask element of the support comprising a core material.
**FIG. 22** depicts a schematic drawing of a cross section through a sheet of thermoplastic material used to form the nose mask element of the support, comprising a core material provided with a first outer (skin contact or facing) layer.
**FIG. 23** depicts a schematic drawing of a cross section through a sheet of thermoplastic material used to form the nose mask element of the support, comprising a core material provided with a first outer (skin contact or facing) layer and a second outer (exterior) layer.
**FIG. 24** depicts a schematic drawing of a cross section through a sheet of thermoplastic material used to form the nose mask element of the support of FIG. 23, further comprising intervening layers.

### DETAILED DESCRIPTION OF THE INVENTION

Unless defined otherwise, all technical and scientific terms used herein have the same meaning as is commonly understood by one of skill in the art. All publications referenced herein are incorporated by reference thereto. All United States patents and patent applications referenced herein are incorporated by reference herein in their entirety including the drawings.

The articles "a" and "an" are used herein to refer to one or to more than one, *i.e.* to at least one of the grammatical object of the article. By way of example, "a fixture" means one fixture or more than one fixture.

Throughout this application, the term "about" is used to indicate that a value includes the standard deviation of error for the device or method being employed to determine the value.

The recitation of numerical ranges by endpoints includes all integer numbers and, where appropriate, fractions subsumed within that range (e.g. 1 to 5 can include 1, 2, 3, 4 when referring to, for example, a number of fixtures, and can also include 1.5, 2, 2.75 and 3.80, when referring to, for example, measurements). The recitation of end points also includes the end point values themselves (e.g. from 1.0 to 5.0 includes both 1.0 and 5.0)

Reference is made in the description below to the drawings which exemplify particular embodiments of the invention; they are not at all intended to be limiting. The skilled person may adapt the device and substitute components and features according to the common practices of the person skilled in the art.

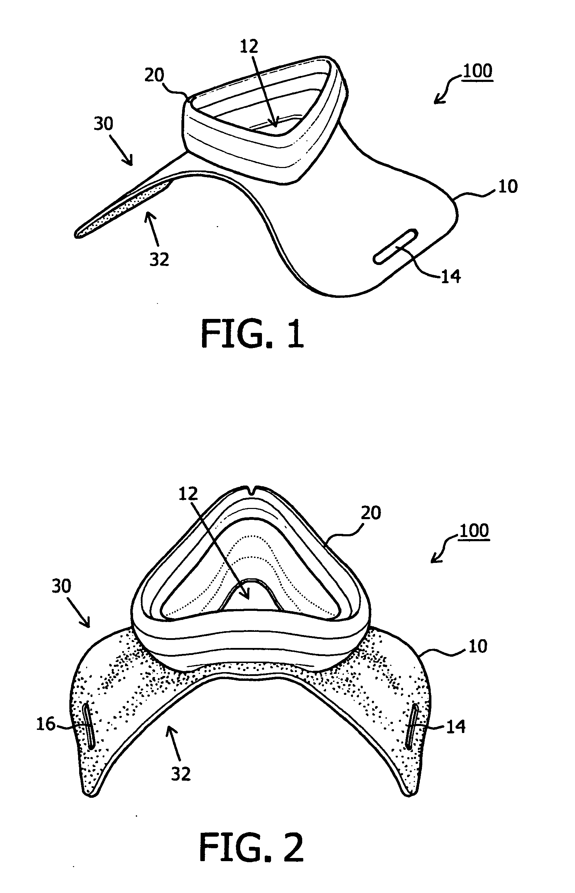

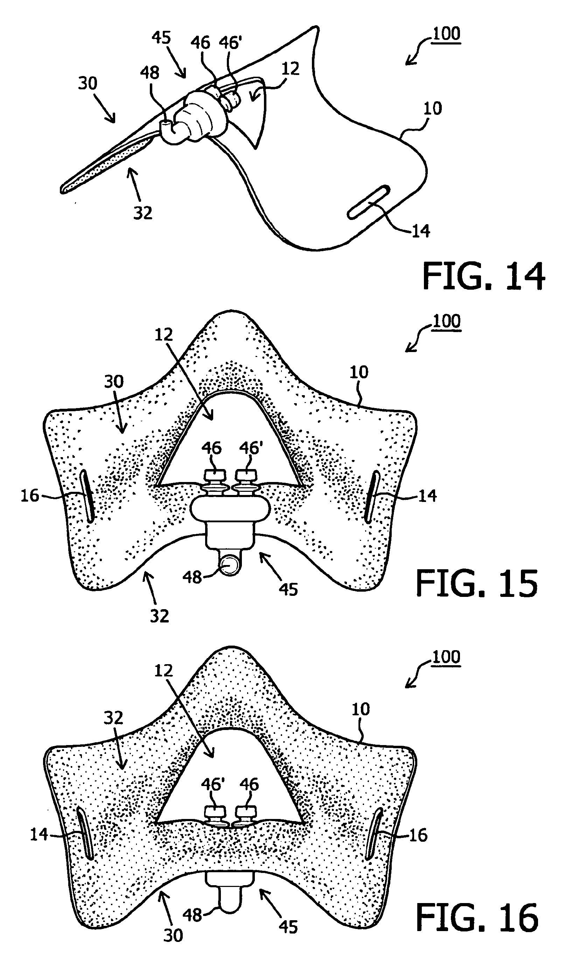

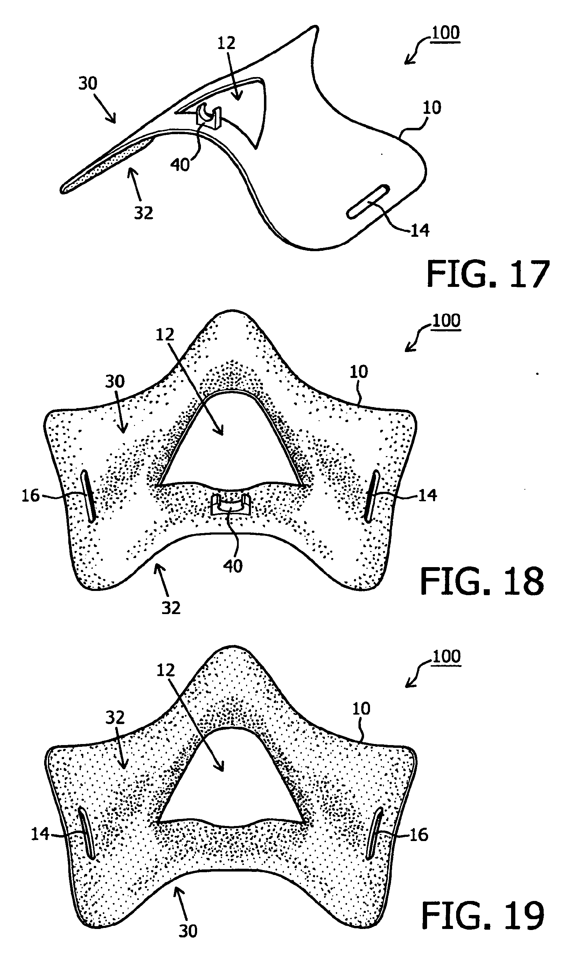

With reference to **FIGs.** 1 to **19****,** the present invention concerns a wearable support **100** formed from a heat moldable nose mask **10** comprising an aperture **12** for the nose, fixtures **14, 16** for attachment to a strap, and a coupling **20, 20a, 40, 45** for attachment of one or more tubes for delivery of the fluid to the nose. The coupling in **FIGs. 1 to 11C** is comprised in a hollow tubular fitting **20, 20a** in connection with the aperture **12** adapted to receive a coupling from an air supply; the coupling in **FIGs. 14 to 16** is comprised in a dual-nozzled adapter **45** having an inlet port for coupling to an air supply; the coupling in **FIGs. 17 to 19** is comprised in a tube clip **40** on the outer surface of the mask **10.**

The nose mask **10** element of the support **100** is heat moldable, meaning it may be adapted to the contours of the face so providing a comfort fit enabling it to be worn for extended periods. Where the support **100** is used for delivery of pressurised air, for instance, in the treatment of sleep apnea, the individual fit also provides a partial sealing function. The skin contact or facing surface **32** of the mask **10** may be lined with a softened material such as a felt, neoprene or knitted material. Alternatively, the support **100** may be supplied with a removable layer which provides the softened effect. The use of a softened material not only provides a pleasant feeling against the skin, but also allows a partial flow of circulating air across rather than through the mask, thereby reducing the build up of perspiration and heat below the mask. The mask **10** is particularly suitable for wearing at night without discomfort, and so may be utilised in the treatment of sleep apnea when the fluid delivered to the noise is air. Equally, the support can be used as an attachment point for a feeding tube or breathing tube inserted through the nose. The support **100** is washable.

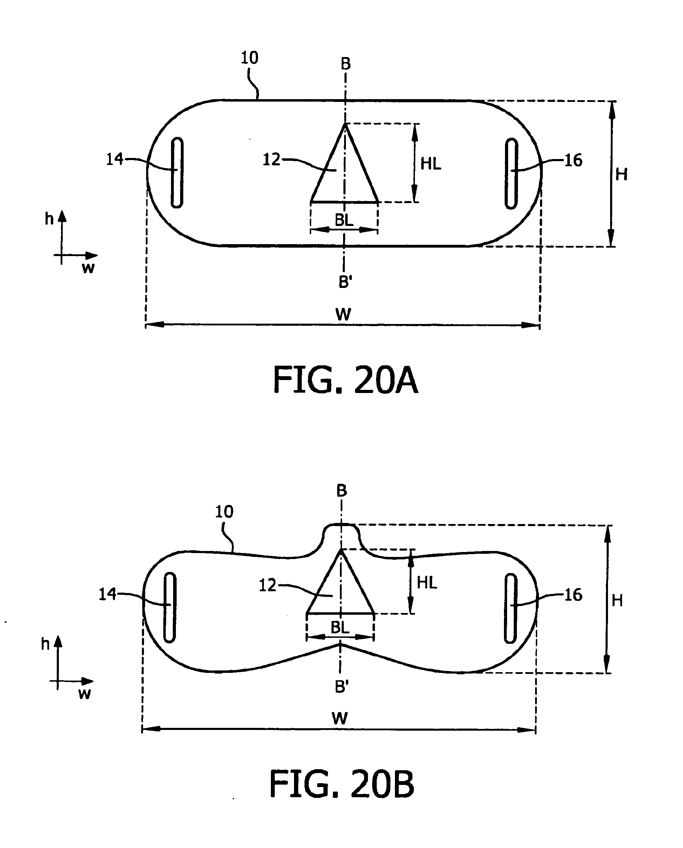

The nose mask **10** element of the support **100** is dimensioned to fit over the nose of the subject. It extends from the nose around at least part of the cheeks, and comprises a pair of fixtures **14, 16** for a strap which passes over the back of the head. The mask **10** is disposed with an aperture **12** for the nose that is preferably essentially triangular in shape, but may alternatively be round, square, oblong or any other suitable shape. It may have the shape substantially of an isosceles trapezium. The shape will be largely determined by the shape of the nose. As will be appreciated, nose mask **10** may be dimensioned so as not to extend over the eyes, mouth or ears. The mask **10** is devoid of apertures for the eyes, mouth or ears. However, an edge of the mask may extend partially around, but not over, the eyes, mouth or ears. For instance, in **FIG. 20B** the upper edge of the mask **10** has a profile that extends partially around the bridge of the nose between the eyes, but it avoid extending over the eyes.

Prior to molding, the nose mask **10** may be flat, or rounded (*e.g.* U-shaped) as shown in **FIGs. 1, 2****,** **3, 4****,** **7****,** **9D****,** **10D****,** **12** and **14** to approximate to the profile of the face. After molding **FIGs. 2****,** **3****,** **15, 16****,** **18** and **19** the mask is adapted to match the facial contours of the subject.

The nose mask 10 element of the support **100** has a substantially longitudinal shape. With reference to **FIGs. 20A** and **20B****,** the mask **10** has a longitudinal (w) direction, and perpendicular thereto, a lateral direction (h). The nose aperture **12** is preferably centrally located along the longitudinal length of the nose mask **10.** The nose aperture **12** may also be centrally located with respect to the lateral height. The nose mask **10** thus comprises two wings which extend from and flank the nose aperture **12.** Each wing is adapted to extend from the nose around at least part of the cheeks. Each longitudinal end of the nose mask is disposed with a fixture **14, 16** for a strap. The mask is configured for wearing such that the longitudinal direction of the mask is oriented essentially parallel to the wearer's eye-line or his left-right axis.

The dimension of the longitudinal width (W, **FIG. 20A****, 20B)** of the nose mask **10** may be defined as the surface distance between the extremities of the opposing longitudinal edges of the mask. The distance is measured in the longitudinal (w) direction. The dimensions of the longitudinal width (W) depend on the characteristics of the subject such as age, shape, and gender, but may be equal to or no more than 15 cm, 16 cm, 17 cm, 18 cm, 19 cm, 20 cm, 21 cm, 22 cm, 23 cm, 24 cm, 25 cm or a value in the range between any two of the aforementioned values, preferably between 16 and 22 cm.

The dimension of the lateral height (H, **FIG. 20A****, 20B)** of the nose mask **10** may be defined as the minimum surface distance between the extremities of the opposing lateral edges of the mask. The distance is measured along the lateral (h) direction. The dimension of the lateral height (H) will depend on the characteristics of the subject such as age, shape, and gender, but may be equal to or no more than 7 cm, 8 cm, 9 cm, 10 cm, 11 cm, 12 cm, or a value in the range between any two of the aforementioned values, preferably between 7 and 12 cm.

The dimension of the longitudinal width (W) is larger than that of the lateral height (H) of the mask. Preferably the dimension of the longitudinal width (W) is 1.5, 2, 2.5, 3, 4, 5, or 6 times larger than that of the lateral height (H), or a value in the range between any two of the aforementioned values, preferably between 1.5 and 3 times.

The aperture **12** for the nose is dimensioned such that the nose substantially passes there through. It is of sufficient size that at least the nostrils pass entirely through the aperture, for instance, during molding. It is of sufficient size that at least the nasal tip passes entirely through the aperture, for instance, during molding. The aperture may be substantially, partly or entirely triangular, round, oblong, square of any other suitable shape. It may have the shape substantially of an isosceles trapezium. The shape will be largely determined by the shape of the nose. Preferably, the aperture is substantially triangular in shape, the triangle having a base that corresponds to the nostril region of the nose. The triangle also has an apex that corresponds to the nasal bone. The orientation of the triangle is such that the base lies in the longitudinal direction (w) of the nose mask **10;** the triangle base does not lie in the lateral direction (h) of the nose mask **10.** Preferably the dimension of the base of the triangle (BL) is 1 cm, 2 cm, 3 m or 4 cm in length. Preferably the perpendicular height of the triangle relative to the base (HL) is 3cm, 4cm, or 5cm in length. Because the aperture allows the nose to pass through, the support avoids that securing pressure is applied to the nose which would otherwise cause discomfort, particularly to the nasal sinuses.

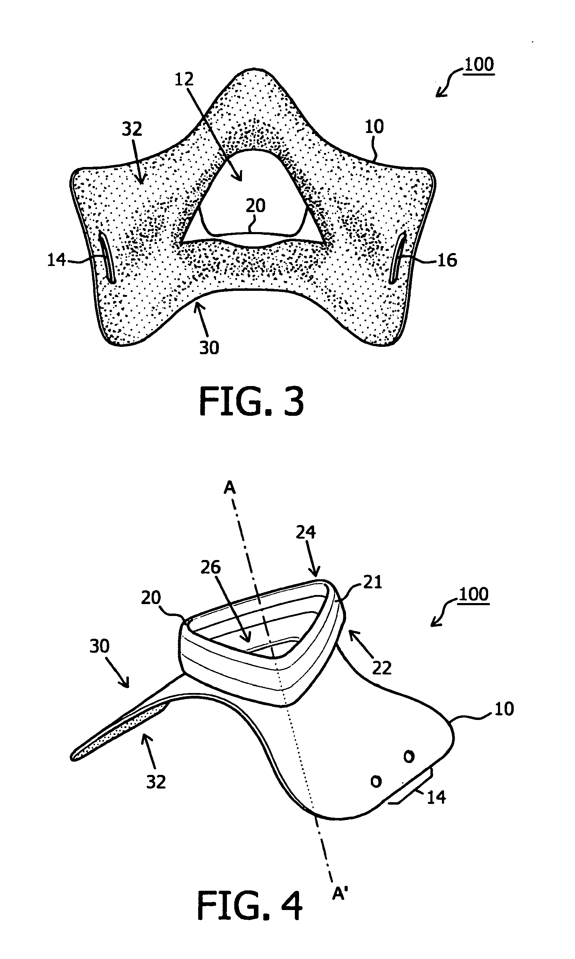

The nose mask **10** may be flat or at least partly curved in the unmolded condition. The above-mentioned dimensions are most preferably obtained when the mask is in the flattened condition, though it will be appreciated that a curved mask can also be measured by transforming a digital model to a flattened state or by measuring along its surface. The curvature may be centered on the aperture **12.** As shown in **FIGs. 1** and **4** the curvature may be around the central lateral axis **B-B' (****FIGs. 20A** and **20B****).** Preferably, the face mask in the lateral direction (h) remains flat in the unmolded condition.

The nose mask **10** is dimensioned to extend from the nose and over the cheek bones. The opposing longitudinal ends of the mask **10** are each provided with a fixture **14, 16** for a strap; because the longitudinal shape positions the strap effectively at the side of the head, and not on the face, the strap does not cut into the face when securing pressure is applied unlike masks of the art. This allows the strap to be worn for prolonged time periods without facial damage, or unwarranted reaction against the strap material. Moreover, the face mask **10** in the molded condition has a large and even area in contact with the surface of the face. Pressure exerted by the strap is evenly distributed, without pressure spots that can lead to an adverse reaction.

The fixtures **14, 16** for the strap may be a pair of slots as depicted, for instance, in **FIGs. 1** to **4****,** **9D****,** **10D****,** **12****,** **14** to **19****,** however, it is not necessarily limited thereto. The fixtures **14,** 16 may comprise one part of a Velcro strip, or one part of a press-stud or snap-fastener, which attaches to a reciprocating element on the strap. Alternatively, each fixture **14, 16** may comprise a pair of holes as indicated, for example, in **FIG. 4****.**

The nose mask **10** may be provided with a plurality of aeration apertures that serve to increase the flow of air to the skin and thereby reduce the build up of perspiration and heat below the mask. The apertures also serve to reduce the weight of the support **100.** The aeration apertures may connect the skin contact or facing surface **32** of the mask with the exterior surface **30** *i.e.* pass from one surface of the mask to the other. Alternatively, the aeration apertures may pass only partially through the mask, for instance through the core and optional intervening layers thermoplastic elements but not through the first outer layer and/or second outer layer (see below). Preferably, the aeration apertures may comprise circular holes; they may have a diameter of 0.5 mm, 1 mm, 1.5 mm, 2.0 mm, 2.5 mm, 3 mm, 3.5 mm, 4 mm, or 5 mm or a value in the range between any two of the aforementioned values; the holes may be present in a region of mask **10** at a density of between 0.5 and 5 holes per cm². The aeration apertures may alternatively or in addition, comprise slots; they may have a length of 1 cm, 2 cm, 3 cm, 4 cm, 5 cm or 6 cm or a value in the range between any two of the aforementioned values, and a width of 0.5 mm, 1 mm, 1.5 mm, 2.0 mm, 2.5 mm, 3 mm, 3.5 mm, 4 mm or 5 mm or a value in the range between any two of the aforementioned values. The aeration apertures may be restricted to the region of the wings.

The nose mask **10** is made from a thermoplastic sheet material **200** deformable under the application of heat to conform to the contours of the face of a subject, which after cooling down retains' the deformed shape and becomes rigid or semi-rigid. The nose mask comprises a thermoplastic composition containing polycaprolactone and polyurethane. According to one embodiment, one or both surfaces of the thermoplastic sheet material **200** is unlined. According to one embodiment, at least one surface is lined with a material such a felt, neoprene, or a knitted fabric that provides a comfortable feeling against the skin.

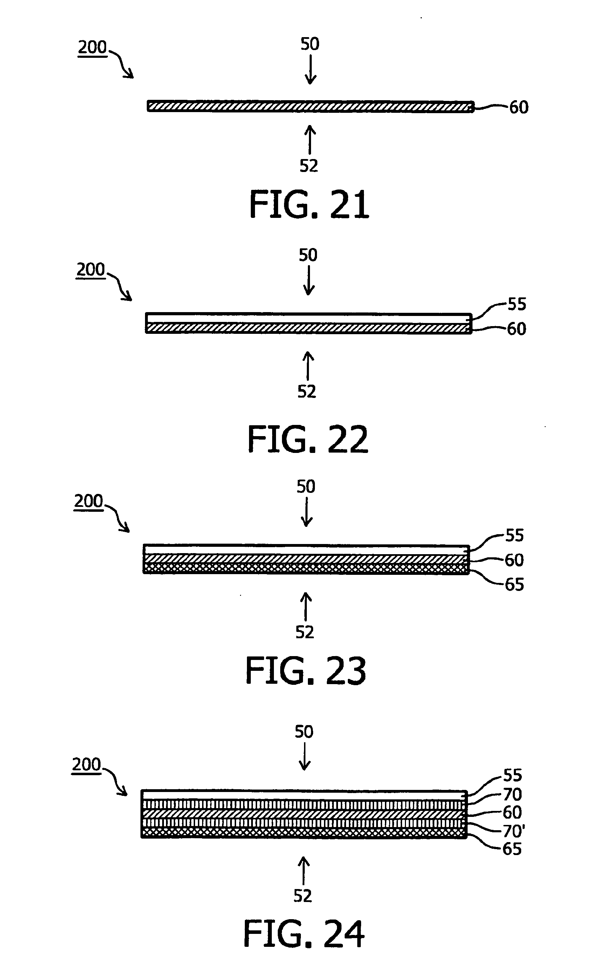

According to one embodiment of the invention, and with reference to **FIG. 21****,** the sheet material **200** comprises a core layer **60** having a first **50** (skin facing 32) side and second **52** (exterior surface **30)** side. The core layer **60** may be provided with a first outer layer **55** disposed over the first **50** side surface of the core layer **(****FIG. 22****)** - which layers are bonded so as to form a single sheet **200.** Alternatively, the first layer may be absent **(****FIG. 21****)** in which case the support **100** may be supplied with a removable layer, that is a sheet of soft material applied to the skin facing **32** side of the nose mask **10** and which gives the support **100** a comfortable feeling against the skin. The removable layer has a shape profile essentially the same as that of the mask **10.** The removable layer is disposable or washable. It may be formed from a woven or non-woven material. The removable layer may be formed from a material that is gas and vapour permeable. Where it is not, it may be provided with the aforementioned aeration apertures, or with a plurality of perforations to increase breathability. The perforations may have a diameter of 0.1 mm, 0.2 mm, 0.3 mm, 0.4 mm, 0.5mm, 0.6 mm, 0.7 mm, 0.8 mm, 0.9 mm, or 1 mm or a value in the range between any two of the aforementioned values.

According to another embodiment of the invention as depicted in **FIG. 23****,** the sheet material **200** preferably comprises at least three separate layers - a core layer **60** having a first **50** (skin facing **32)** side and second **52** (exterior surface **30)** side, a first outer layer **55** disposed over the first **50** side surface of the core layer and a second outer layer **65** disposed over the second **52** side surface of the core layer - which layers are bonded so as to form a single sheet **200.**

One or more intervening layers **70, 70' (****FIG. 24****)** may be disposed between the core layer **60** and the first outer layer **55,** and/or disposed between the core layer **60** and the second outer layer **65.**

The overall thickness of the sheet **200** may be 1.5 mm, 1.6 mm, 1.7 mm, 1.8 mm, 1.9 mm, 2.0 mm, 2.2 mm, 2.4 mm, 2.5 mm, 2.6 mm, 2.7 mm, 2.8 mm, 2.9 mm, 3.0 mm, 3.2 mm, 3.4 mm, 3.5 mm, 3.6 mm, 3.7 mm, 3.8 mm, 3.9 mm, or 4.0 mm or a value in the range between any two of the aforementioned values.

The sheet **200** is distortable at temperatures of between 50 and 70 deg Celsius, depending on the polycaprolactone content. Typically the sheet is brought into the malleable condition by heating in a water bath set around 5 degrees Celsius above the melting temperature, most preferable at 65 deg Celsius. It alternatively, be heated using a convection or fan oven, or a microwave oven. An impression of the face, particularly in the cheek region around the nose, is taken by placing the first **50** side *i.e.* skin contact or facing side **32** in contact with the skin and/or hair, and applying pressure to the sheet **200.** After molding, the sheet **200** is allowed to cool, hardening in the process. A resulting nose mask **10** is shown in **FIGs. 2****,** **3****,** **15, 16****,** **18** and **19****.** It is noted that the shape of the mask **10** may be adjusted by the application of hot air to the exterior surface 30; this allows the shape to be fine-tuned, for instance, to relieve pressure points experienced by the wearer. Hot air may be provided by a hot-air blower (e.g. a hairdryer).

The sheet **200** exhibits excellent deformability properties, conforming to the shape of the face without the need to apply excessive pressure. The first layer **55** where present provides a comfortable wearing against the skin, and prevents adhesion to the skin and/or hair by the core layer **60.** It also insulates the heat emitted by the heated core **60** layer from the skin of the subject. The second layer **65** where present prevents the core **60** from adhering to itself or other articles, and in addition reduces the hardening time. The sheet is considerably thinner than conventional masks, it is semi-rigid, has a soft touch feeling, and, therefore, provides a high degree of comfort in both molding and wearing.

The core layer **60** of a multilayer sheet material comprises a thermoplastic composition containing polycaprolactone and polyurethane. It has a thickness between 1 mm, 1.5 mm, 2.0 mm, 2.5 mm, 3 mm or a value in the range between any two of the aforementioned values, preferably between 1 mm 3 mm.

The polyurethane may present in an amount of 0%, 10%, 20 %, 30 %, 40 % or 50% (w/w), or a value in the range between any two of the aforementioned values, preferably 20 to 40 %, most preferably 30%. The polycaprolactone may be present in an amount of 60 %, 70 %, 80 %, or 90% (w/w) or a value in the range between any two of the aforementioned values, preferably 60 to 80 % (w/w), most preferably 70%. Typically, there will be more polycaprolactone than polyurethane which polycaprolactone lowers the temperature at which the sheet deforms. The ratio of polycaprolactone:polyurethane is (w:w) may be 5:1, 4:1, 3:2, 3:1, 2.3:1, 2:1 preferably 2.3:1.

The molecular weight of the polyurethane may be equal to or less than 10 000, 20 000, 30 000, 40 000, 50 000, 60 000, 70 000, 80 000, 90 000, 100 000, 120 000, 140 000, 150 000, or a value in the range between any two of the aforementioned values, preferably between 10 000 and 100 000. Polyester polyurethane is the preferred polyurethane.

The molecular weight of the polycaprolactone may be 10 000, 20 000, 30 000, 40 000, 50 000, 60 000, 70 000, 80 000, 100 000, 200 000, 300 000, 400 000, 500 000, or a value in the range between any two of the aforementioned values, preferably between 10 000 and 60 000, more preferably between 37 000 and 500 000.

Caprolactone polyester polyurethane is particularly suitable, which polyurethane may be obtained by reacting isocyanate and polycaprolactone-based polyester. Such a caprolactone polyester polyurethane is commercially available as a granulate. The melting point of said polycaprolactone polyester polyurethane lies between 190 and 210 degrees Celsius. By adding the polycaprolactone, also preferably in granulate form, a thermoplastic composition is obtained that is distortable and kneadable at a temperature of - 69 degrees Celsius and remains distortable by cooling down about 50 degrees Celsius. At this temperature, the core layer **60** may be stretched at least up to twenty times the original length thereof. In the hardened condition, the thermoplastic composition is semi-rigid and has a memory effect that, after heating, returns to the shape formed on cooling. It is non-elastic in the hardened condition.

Advantageously, the core layer **60** comprises between 1 to 40 % (w/w) of microspheres of non-metallic, heat-accumulating material which is especially suited for heating in a microwave oven. Preferred are glass microspheres with a diameter between 20 and 800 micrometer. A colouring agent may be added to the core.

One or more intervening layers **70, 70' (****FIG. 24****)** may be disposed between the core layer **60** and the first outer layer 55, and/or disposed between the core layer **60** and the second outer layer **65.**

Typically an intervening layer **70, 70'** comprises a higher polycaprolactone content compared with the core layer **60,** and thus can be deformed at a lower temperature than the core layer **60.** According to one aspect of the invention, an intervening layer **70, 70'** has a polycaprolactone content that is higher than the core layer **60** by 5 %, 10 %, 15 %, 20 %, 25 %, 30 %, 50 %, 100 %, 200 %, 300 %, or 400 % or a value in the range between any two of the aforementioned values, preferably between 100 % and 400 %. An intervening layer **70, 70'** may be comprised of pure polycaprolactone.

Thickness of an intervening layer **70, 70'** may be 20%, 30%, 40%, 50%, 60%, 70% or 80% that of the core layer **60** or a value in the range between any two of the aforementioned values, preferably between 40 % and 60 %.

By virtue of an intervening layer **70, 70',** the sheet 200 can be deformed at a lower temperature, since only the only the intervening layer **70, 70'** of the sheet **100** needs to be heated above the temperature at which the thermoplastic material deforms; the result is that heating up time before use is shorter and comfort to the subject is improved. As the core layer **60** has not to be brought at such high temperature, the expansion of the core **60** is also smaller. Due to expansion when heating, successive windings could be pressed so strongly together that they adhere strongly to each other.

In an advantageous embodiment of the invention, the intervening layer **70, 70'** comprises between 1 to 40 % (w/w) of microspheres of non-metallic, heat-accumulating material which is especially suited for heating in a micro-wave oven. Preferred are glass microspheres with a diameter between 20 and 800 micrometer. A colouring agent may be added to the intervening layer **70, 70'.**

The first outer layer **55,** also referred to as the 'first layer' herein is a body-side liner *i.e.* the skin contact or facing layer. It may be made from any material that is inert and provides a non-irritating and comfortable feeling against the skin such as felt, flocking material, neoprene or a knitted material.

When the first outer layer **55** is neoprene, it may be neoprene or an elasticated neoprene. It may be elasticated by virtue of a layer of elastic material on one side. The neoprene may be disposed with a plurality of perforations to increase breathability. The perforations particularly assist circulation when aeration apertures are limited to the layers of the thermoplastic material and do not extend through to the first outer layer **55** and optionally second outer layer **65.** The perforations may have a diameter of 0.1 mm, 0.2 mm, 0.3 mm, 0.4 mm, 0.5mm, 0.6 mm, 0.7 mm, 0.8 mm, 0.9 mm, or 1 mm or a value in the range between any two of the aforementioned values. Such perforations assist, for example, with heating the core layer **60** by providing access directly to the core, and with drainage when heating the sheet occurs in a warm water bath.

When the first outer layer **55** is a knitted material, it comprises a polyamide-based knitted fabric material with a thickness outside the core layer **60** of between 0.05 and 1.5 mm. According to one aspect of the invention, the knitted fabric material is formed from a yarn comprising between 80 % to 95 % polyamide, and between 5 % and 15 % elastane, preferably comprising 90 % polyamide and 10 % elastane. The fabric weight may be 190 g/m², 200 g/m², 210 g/m², 220 g/m², 230 g/m², or 240 g/m², preferably between 210 g/m² and 230 g/m². The fabric may be coloured, for example, with a calming colour such as a neutral tone, pastel shade, or primary colour such as blue.

When the first outer layer **55** is a flocking material, the material may be any finely cut natural or synthetic fiber. It is applied so as to produce a velvet finish. The flock may be coloured, for example, with a calming colour such as a neutral tone, pastel shade, or primary colour such as blue.

The thickness of the first outer layer **55** is equal to or less than 0.025 mm, 0.05 mm, 0.06 mm, 0.08 mm, 0.1 mm, 0.5 mm, 0.8 mm, 1 mm, 1.2 mm, 1.5 mm or a value in the range between two of the aforementioned values, preferably between 0.05 and 1.5 mm, more preferably having a thickness of between 0.1 and 0.4 mm.

The core **60** is bonded to the first layer **55** or intermediate **70, 70'** layer by virtue of the adhesive property of the core **60** in the fluid condition. Adhesion may be enhanced pressing the first layer **55** onto the core layer **60,** for example, by the use of roller during manufacture.

Due to the presence of first layer **55,** the sheet **200** may be applied directly in distortable condition on the skin. It does not adhere to hair and skin. It does not either leave visible fingerprints, so that disposable gloves not necessary for the application. In addition, the first layer **55** forms a thin insulating layer, such that the skin is not subject to elevated temperatures that might otherwise harm such areas.

The first layer **55** is the skin-contact surface of the mask **10** and thus provides a pleasant feeling against the skin, and also allows a flow of circulating air across (rather than through the mask in the absence of aeration apertures), thereby reducing the build up of perspiration and heat below the mask.

The second layer **65,** also referred to as the 'second layer' herein, comprises a soft resilient open cell foam plastic disposed over the core layer **60** and forming contact therewith or with the optional intermediate layer **70, 70'.**

The second layer **65** forms a physical and insulating layer which protects the core layer **60** in the malleable condition. The foam plastic outside the core layer **60** or optional intermediate layers **70, 70',** before the application, does not comprise thermoplastic material as for example the foam plastic from the composite material according to US-A 3,728,206 which foam plastic does not form a coating. The second layer **65** forms a covering which prevents portions of the sheet **200** from adhering inadvertently to one another. The second layer **65** allows the sheet to be handled with bare hands without, damage to the core **60** or optional intermediate layers 70, **70',** and without adhering to the fingers.

The thickness of the second outer layer **65** is equal to or less than 0.05 mm, 0.06 mm, 0.08 mm, 0.1 mm, 0.5 mm, 0.8 mm, 1 mm, 1.2 mm, 1.5 mm or a value in the range between two of the aforementioned values, preferably between 0.05 and 1.5 mm, more preferably having a thickness of between 0.4 and 0.6 mm. It has been found that layers of foam plastic with a thickness outside of the core of more than 1.5 mm would prevent a good adherence to the underlying layer (*i.e*. core or intermediate layer) when the sheet is deformed.

The second layer **65** is made from a material able to withstand temperatures at which the core is softened or weakened. They are preferably made from a non-thermoplastic plastic, such as polyurethane formed into soft, open cell foam. The foam that has such an open-cell structure that core layer **60** in softened condition can traverse it when pressure is exerted but also that the second layer **65** can be elastically deformed without tearing. When the sheet **200** is heated, it can be stretched up to four times its original length without breakage of the second layer **65.** Suitable foam plastics for the second layer **65** are polyurethane, particularly polyester polyurethane and polyether foam.

The core **60** is bonded to the second layer **65** or intermediate 70, **70'** layer by virtue of the adhesive property of the core **60.** Adhesion may be enhanced pressing the second layer **65** onto the core layer **10,** for example, by the use of roller during manufacture.

For some applications, the second layer **65** may be provided with perforations (not shown) with a diameter of at least 0.5 mm and for example 2 mm. Such perforations assist, for example, with heating the core layer **60** by providing access directly to the core, and with drainage when heating the sheet occurs in a warm water bath.

Cross-wise through core **60,** first 55 and second **65** layers, smaller perforations with a diameter of at least 0.5 mm and preferably about 1 to 1.3 mm may be provided, so as not to hamper the skin breathing after applying the sheet. Said perforations may lie on rows crossing each other under 90 degrees and making an angle of 45 degrees with the transversal direction of the sheet of composite material, at a distance of each other in the rows of 1.5 to 4 mm. The skin may still breathe even after application of the material.

The support is provided with a pair of strap fixtures **14, 16,** adapted to affix a strap that passes around the back of the head, and secures the mask **10** to the face of the subject. The fixtures **14, 16** for the strap may be a pair of slots as depicted, for instance, in **FIGs. 1** to **3****,** **9D****,** **10D****,** **12****,** **14 to 19****,** however, it is not necessarily limited thereto. The fixtures **14, 16** may comprise one part of a Velcro strip, or one part of a press-stud or snap-fastener, which attaches to a reciprocating element on the strap. Each fixture **14, 16** may alternatively comprise a pair of holes as indicated, for instance, in **FIG.** 4. The strap is preferably made from an elasticated substance, such as a strip of elasticated material, and may include a length adjuster, to adjust the length of the strap and thus pressure exerted by the mask **10** on the face.

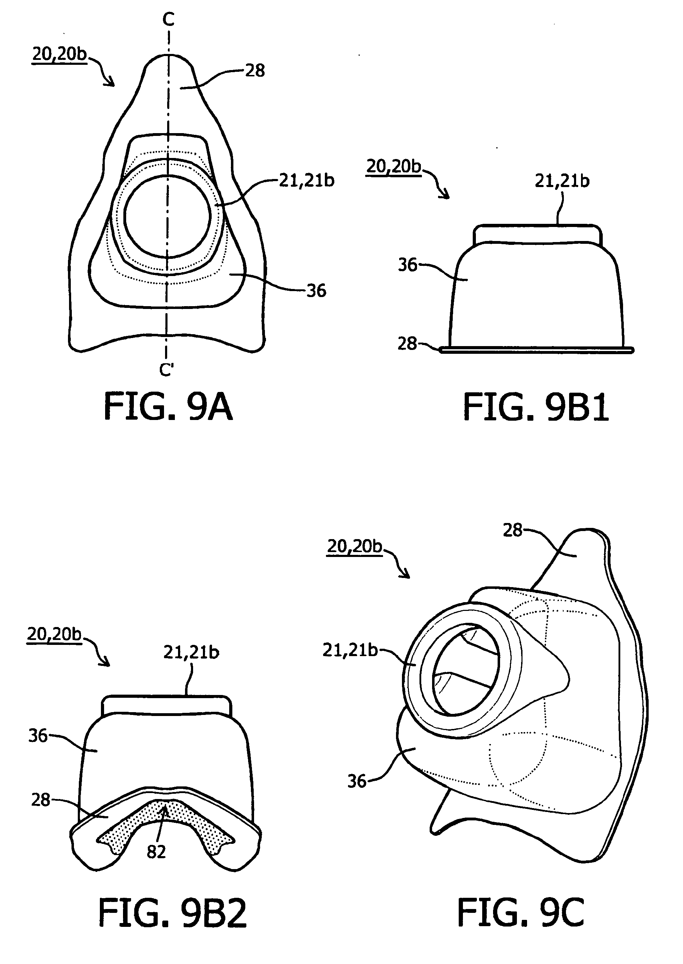

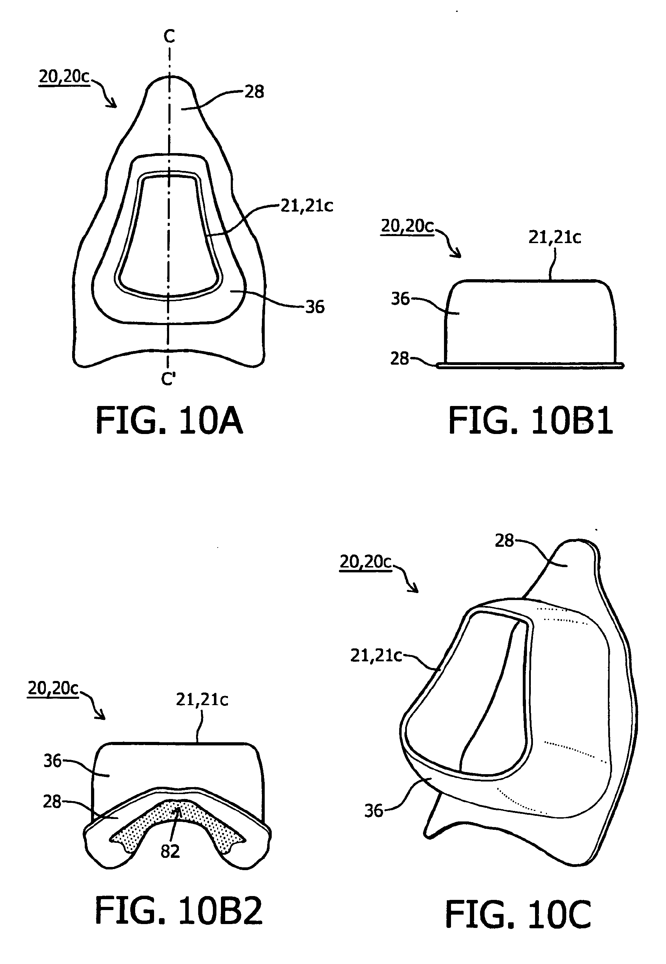

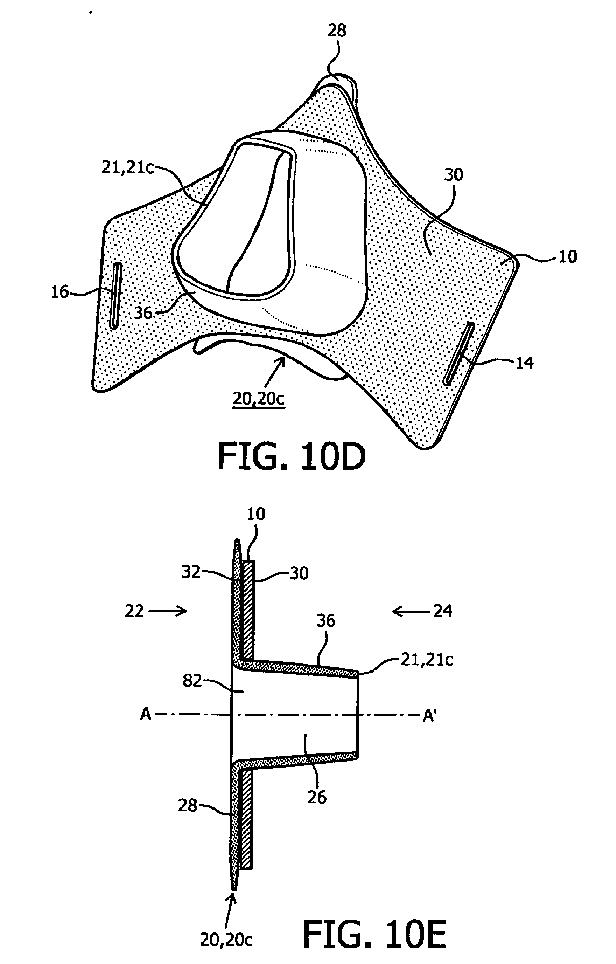

The support **100** also includes one or more couplings **20, 20a-c, 40, 45** for a tube such as a catheter for the delivery of fluid to the nose of the subject. The support **100** maintains the position of the tube relative to the face, permitting the subject to receive treatment for prolonged periods while being able to move the head freely. The support 100 prevents the tube from inadvertently disconnecting from the nose. The coupling **20, 20a-c, 40, 45** may be permanently or dismountably fixed (e.g. **20a-c)** to the mask **10.** According to one embodiment of the invention **(****FIGs.** 1 **to 9****),** the coupling is comprised in a hollow tubular fitting **20, 20a-c,** provided at one (proximal) end with an opening **82** for receiving the nose, and at the other (distal) end with an inlet port **21** for coupling to a fitting for providing gas (e.g. air). The proximal end is in connection with the nose aperture **12** of the mask **10.** The tubular fitting **20, 20a-d** may be essentially longitudinal as shown in **FIG. 4****,** having a longitudinal (central) axis **A-A'** between the proximal **22** and distal **24** ends. It is provided with a passageway **26** suitable for the passage of fluids, that connects the inlet port **21** to the opening **82** at the proximal **22** end. As shown in **FIG. 4****,** the port **21** may have an essentially triangular profile when viewed the along the **A-A'** axis towards the proximal **22** end, making it suitable for attachment to existing triangular surgical couplings for standard nose masks. The triangular shape may be evident as an isosceles trapezium as shown, for example, in **FIGs. 9C** and **10C****.** According to one embodiment of the invention, the port **21** is adapted to receive a triangular profile gas coupling for a standard nose mask.

The terms "distal" and "proximal" are used through the specification, and are terms generally understood in the field to mean towards (proximal) or away (distal) from the wearer. Thus, "proximal" or "proximal side" means towards the wearer. Conversely, "distal" or "distal side" means away from the wearer.

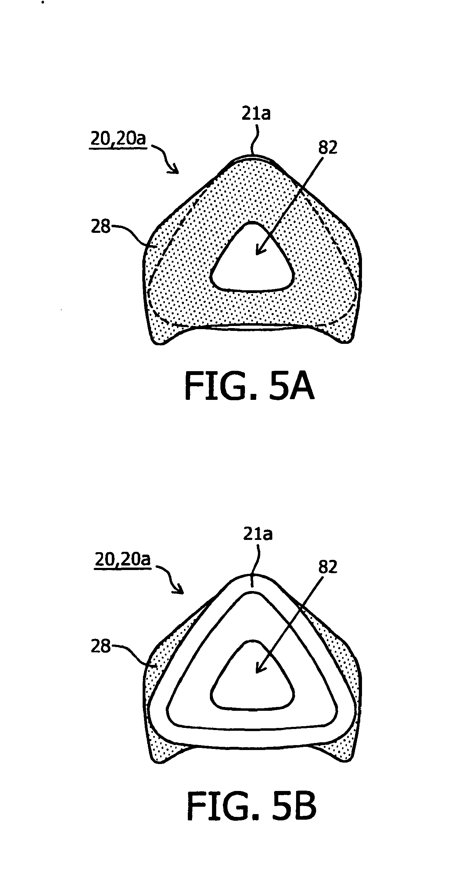

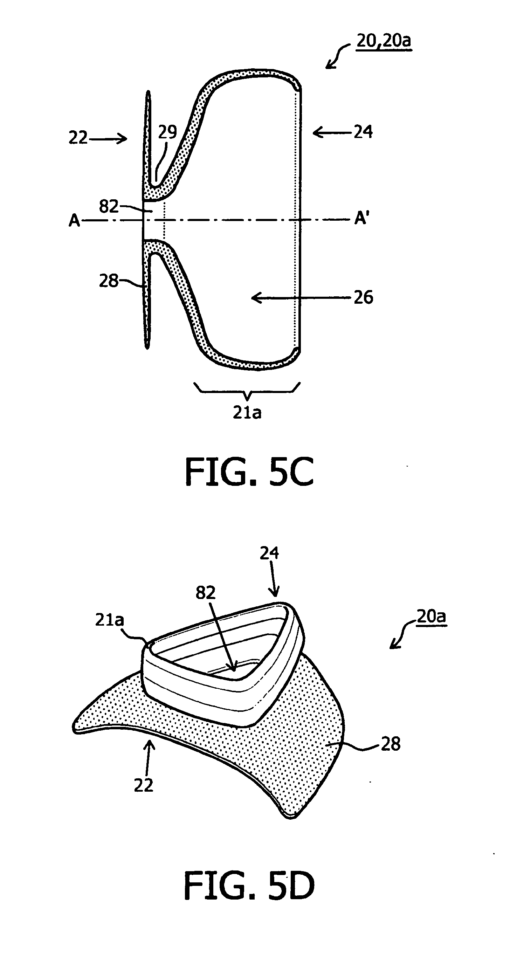

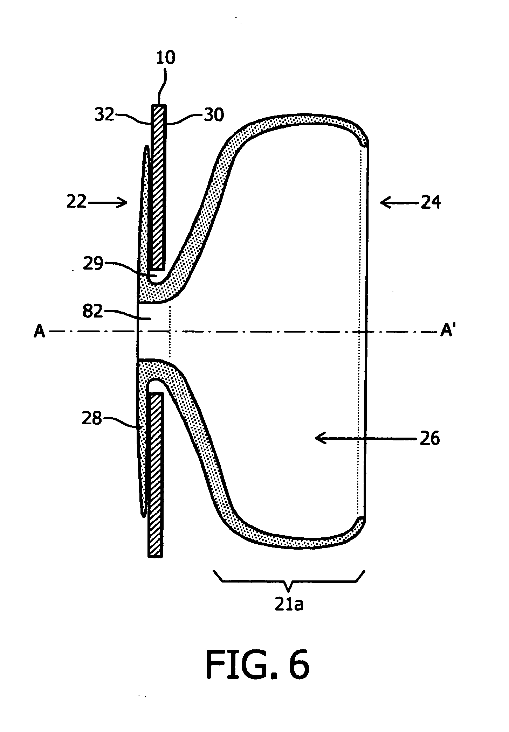

According to one embodiment of the invention, the coupling is a tubular fitting **20a** configured for dismountable attachment to the nose mask **10.** The dismountable attachment may be achieved by any means. Preferably, the proximal end **22** of the dismountable tubular fitting **20a** is extended with an open, flexible flange 28, **(****FIGs. 5A****, B, C, D)** which flange **28** is configured to pass through the nose aperture **12** in the mask **10** in a folded condition such that the flange **28** can be disposed on the skin-facing side **32** of the nose mask **10 (****FIG. 6****),** while the inlet port **21a** disposed on the exterior side **30** of the mask **10,** thereby dismountably attaching the removable coupling **20a** to the nose mask **10.**

More in particular, the tubular fitting 20a may be essentially longitudinal as shown in **FIG. 5C****,** having a longitudinal (central) axis **A-A'** between the proximal **22** and distal **24** ends. It is provided with a flange **28** around the opening **82** at the proximal **22** end, the inlet port **21a** at the distal end **24,** and a passageway **26** connecting the inlet port **21a** to the flanged opening **82.** The flange **28** has a proximal **22** (skin facing) side and a distal **24** (mask facing) side. The opening **82** is essentially central to the area of the flange, and adapted to receive the nose.

Attachment of the tubular fitting **20a** provided with the flanged **28** opening **82** is effected by advancing the flexible flange **28** in a folded state through the nose aperture **12** of the nose mask **10.** Having passed through the nose aperture **12** from the exterior side **30,** the flexible flange **28** unfolds and abuts the skin-facing **32** side of the mask **10.** The inlet port **21a** is disposed on the exterior side **30** of the mask **10,** so sandwiching or suspending the mask **10** between the flange **28** and the port **21 a,** thereby dismountably attaching the removable coupling **20a** to the nose mask **10.** As such, the flange **28** has an unfoldable property that allows folding for passage through the nose aperture **12,** and unfolding once *in situ* abutting the skin-facing **32** side of the mask **10.**

The flange **28,** besides providing a gas-sealing effect against the skin, also gives a comfortable feeling, and, having a mechanical buffering property, can mitigate frictional abrasive or indent effects the rigid nose mask **10** may impose during wearing. The removable coupling **20a** provided with the flange **28** can dismounted and replaced after regular intervals or after sufficient wear and tear, so reducing the build up of dust and allergens; the lifespan of the wearable support **100** is thereby prolonged. As the flange is flexible, its shape is adapted to the molded form of the nosemask **10** and it need not have any thermoplastic properties. It is anticipated that different sizes of flange **28** would be available to suit different sizes and shapes or nose mask.

The flange **28** of the dismountable coupling **20a** forms the proximal **22** wall of a groove **29** configured to seat the inside edge of the nose aperture **12.** The groove **29** is preferably ring-shaped. The groove interior is preferable aligned essentially perpendicular to central **A-A'** axis of the fitting **20a.** The groove **29** is disposed along the circumference of the flange **28.** The flange **28** is preferably oriented essentially perpendicular to the longitudinal **A-A'** axis of the dismountable tubular fitting **20a.** The groove **29** preferably has a triangular profile when viewed along the **A-A'** axis towards the distal **24** end. The groove **29** preferably has a triangular profile when viewed along the **A-A'** axis towards the distal **24** end. The triangular profile of the groove **29** reciprocates the triangular profile of the nose mask **10** aperture **12.** The triangular profile of the groove **29** may be smaller in size than that of the port **21** a.

As mentioned above, the flange **28** of the dismountable coupling **20a** forms the proximal **22** wall of a groove **29;** the distal **24** (other) wall of the groove **29** may be formed from the back (proximal) wall of the port **21 a** as shown in **FIG. 5C** and **6****.**

As shown in **FIG.. 6** the flange **28** adjoining the groove **29** is adapted to flank the sub-region of thermoplastic material disposed around the nose mask **10** aperture **12** on the skin-facing **32** surface, thereby sealing the dismountable tubular fitting **20a** against the nose mask **10.**

The flange **28** is preferably formed from any suitable material exhibiting the requisite unfoldability property *i.e.* the ability to be folded and unfolded without weakening or other damage. Preferably, the coupling is formed from a flexible material such as silicone.

The flange **28** may have any profile when viewed along the **A-A'** axis towards the distal **24** end, but in general, it will at least partially correspond with the shape of the nose mask **10.** The profile refers to the outer shape, or the profile of the opening **82,** or both. By correspond, it is meant that they essentially overlap, but some deviation is permitted, for example, the profile of the flange **28** may differ in area compared with the profile of the nose mask **10** by 0%, ±10%, ±15%, ±20%, ±25 % or a value between any two of the aforementioned values.

While the profile of flange **28** at least partially corresponds with that of the nose mask **10** according to one embodiment, an independent description of the profile of the flange **28** now follows. The flange **28** is dimensioned to fit around the nose of the subject. It preferably extends from the nose around at least part of the cheeks. The flange **28** is disposed with an opening **82** for the nose that is preferably triangular in shape, but may alternatively be round, square, oblong or any other suitable shape. The shape will be largely determined by the shape of the nose. As will be appreciated, the flange **28** may be dimensioned so as not to extend over the eyes, mouth or ears. The flange **28** is devoid of apertures for the eyes, mouth or ears. However, an edge of the flange **28** may extend partially around, but not over, the eyes, mouth or ears.

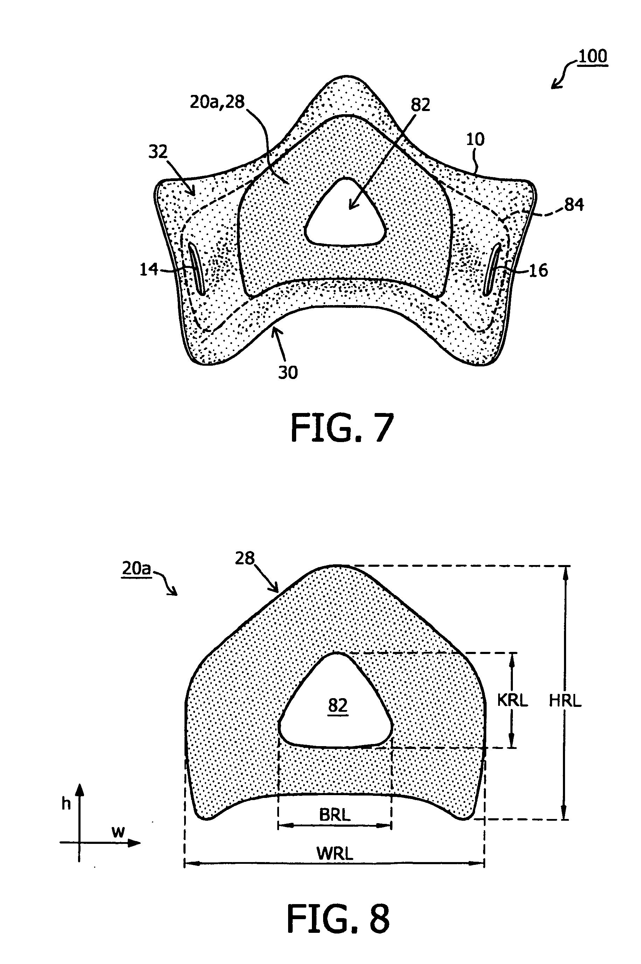

The flange **28** may have a substantially longitudinal shape. With reference to **FIG. 8****,** the flange **28** has a longitudinal (w) direction, and perpendicular thereto, a lateral direction (h). The longitudinal (w) direction is aligned with the base of the nose (*i.e*. parallel to the eyes). The opening **82** is preferably centrally located along the longitudinal length of the flange **28.** The opening **82** may also be centrally located with respect to the lateral height. The flange **28** thus comprises two wings which extend longitudinally from and flank the opening **82.** Each wing is adapted to extend from the nose around at least part of the cheeks.

The flange **28** may be planar or flat in the native state, as shown for instance in FIG. **9B1.** According to an alternative embodiment, the flange may be curved in the native state as shown, for example, in **FIG. 9B2.** The curvature may be around the central lateral axis (C-**C').** The curvature provides a closer fit of the flange against skin.

It is within the scope of the invention that the outer shape of the flange **28** is identical to that of the nose mask **10,** or corresponds at least partially to that of the nose mask 10. The outer shape of the flange **28** may at least partially extend beyond the profile of the nose mask **10** or *vice versa.*

The dimension of the longitudinal width (WRL) of the flange **28** may be defined as the surface distance between the extremities of the opposing longitudinal edges of flange **28.** The distance is measured in the longitudinal (w) direction. The dimensions of the longitudinal width (WRL) will depend on the profile of the nose mask **10,** and may differ by 0%, ±10%. ±15%, ±20%, ±25 % or more.

The dimension of the lateral height (HRL) of the flange **28** may be defined as the minimum surface distance between the extremities of the opposing lateral edges of the mask. The distance is measured along the lateral (h) direction. The dimensions of the lateral height (HRL) will depend on the profile of the nose mask **10,** and may differ by 0%, ±10%, ±15%, ±20%, ±25 % or more.

The dimension of the longitudinal width (WRL) is larger than that of the lateral height (HRL°) of the flange **28.** Preferably the dimension of the longitudinal width (WRL) is 1.5, 2, 2.5, 3, 4, 5, or 6 times larger than that of the lateral height (HRL), or a value in the range between any two of the aforementioned values, preferably between 1.5 and 3 times.

The opening **82** for the nose is dimensioned such that the nose substantially passes there through. It is of sufficient size that at least the nasal tip passes entirely through the opening **82.** The opening **82** may be triangular, round, oblong, square of any other suitable shape. It may have the shape substantially of an isosceles trapezium. The shape will be largely determined by the shape of the nose. Preferably, the opening **82** is substantially triangular in shape, the triangle having a base that corresponds to the nostril region of the nose. The triangle also has an apex that corresponds to the nasal bone. The orientation of the triangle is such that the base lies in the longitudinal direction (w) of the flange **28;** the triangle base does not lie in the lateral direction (h) of the flange **28.** Preferably the dimension of the base of the triangle (BRL) is 1 cm, 2 cm, 3 m or 4 cm in length. Preferably the perpendicular height of the triangle relative to the base (KRL) is 3cm, 4cm, or 5cm in length. The profile of the opening **82** of the flange **28** may differ in area compared with the profile of the aperture **12** of the nose mask **10,** by 0%, ±10%, ±15%, ±20%, ±25 % or more.

The thickness of the flange **28** will generally be determined by the material used for construction, and by the requisite unfoldability property *i.e.* the ability to be folded and unfolded without damage. However, as a general guidance, the minimum thickness of the flange **28** will be equal to or less than 0.5 mm, 1 mm, 2 mm, 3 mm, 4 mm, or a value in the range between any two of the aforementioned values.

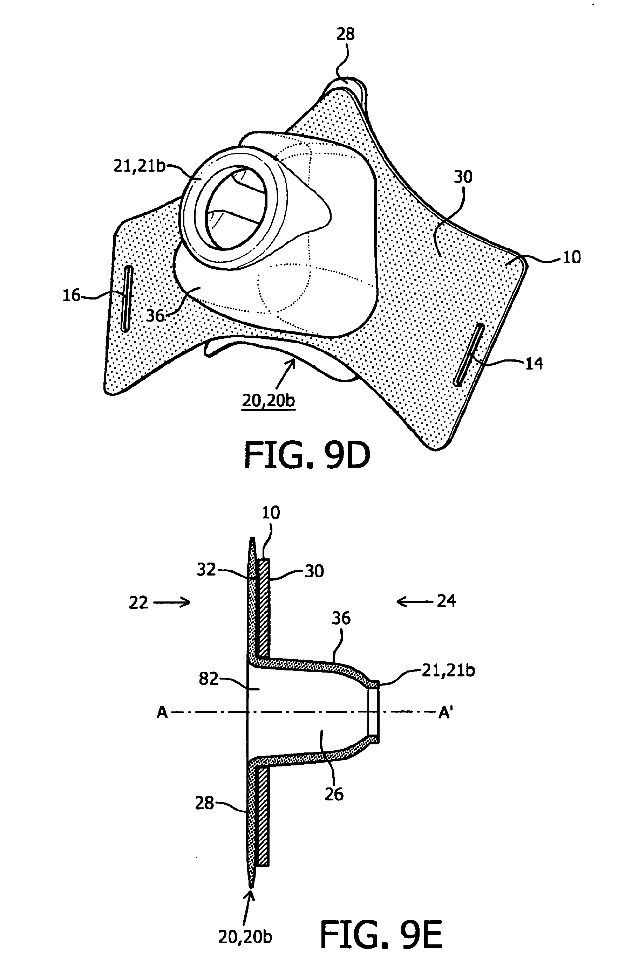

According to one embodiment of the invention, the coupling is a tubular fitting **20b** exemplified in **FIGs. 9A****, 9B1, 9B2, 9C, 9D** configured for dismountable attachment to the nose mask 10. The dismountable attachment may be achieved by any means. Preferably, the proximal end 22 of the dismountable tubular fitting **20b** is provided with an open, flexible flange **28,** at one end, and an inlet port **21, 21b** at the other end **(****FIGs. 9A**, **9B1, 9B2, 9C****,** **9D****).** The inlet port **21** may be circular **21b** as shown in **FIGs. 9A****, 9B1, 9B2**, 9C, **9D****,** and an elongated passageway **26** connects the inlet port **21 b** to the flanged opening **82.** The outer wall **36** of the passageway **26** has an essentially triangular or essentially isosceles trapezium transverse profile the outer profile being the outer profile of a plane perpendicular to the **A-A'** axis. The wall is intact and fluid impermeable to prevent leakage fluid. The inlet port **21 b** and outer wall **36** of the passageway are dimensioned to slidably pass through the nose aperture **12** in the mask **10** without substantial hinderance, such that the flange **28** is disposed on the skin-facing side **32** of the nose mask **10 (****FIG. 9D, 9E),** while the inlet port **21 b** and the substantial part of the passageway wall 36 are disposed on the exterior side **30** of the mask **10,** thereby dismountably attaching the removable coupling **20b** to the nose mask **10.**

More in particular, the tubular fitting **20a** may be essentially longitudinal as shown in **FIG. 9E****,** having a longitudinal (central) axis **A-A'** between the proximal **22** and distal **24** ends. It is provided with a flange **28** around the opening **82** at the proximal **22** end, the inlet port **21b** at the distal end **24,** and a walled **36** passageway **26** connecting the circular inlet port **21b** to the flanged opening **82.** The flange **28** has a proximal **22** (skin facing) side and a distal **24** (mask facing) side. The opening **82** is essentially central to the area of the flange, and adapted to receive the nose.

Attachment of the tubular fitting **20b** provided with the flanged **28** opening **82** is effected by advancing the inlet port **21 b** and passageway **26** through the nose aperture **12** of the nose mask **10.** The passageway is dimensioned to pass through the aperture essentially unhindered. Having passed the inlet port **21b** and passageway **26** through the nose aperture **12** from the skin-facing **32,** the distal side of the flexible flange **28** abuts the skin-facing **32** side of the mask **10.** The inlet port **21** b and substantially all the passageway **26** are disposed on the exterior side **30** of the mask **10,** thereby dismountably attaching the removable coupling **20b** to the nose mask **10.**

The flange **28,** besides providing a gas-sealing effect against the skin, also gives a comfortable feeling, and, having a mechanical buffering property, can mitigate frictional abrasive or indent effects the rigid nose mask **10** may impose during wearing. The removable coupling **20b** provided with the flange **28** can dismounted and replaced after regular intervals or after sufficient wear and tear, so reducing the build up of dust and allergens; the lifespan of the wearable support **100** is thereby prolonged. As the flange is flexible, its shape is adapted to the molded form of the nosemask **10** and it need not have any thermoplastic properties. It is anticipated that different sizes of flange **28** would be available to suit different sizes and shapes or nose mask.

The flange **28** is preferably formed from any suitable material bio-compatible material that exhibits a sealing effect. Preferably, it is formed from a flexible material such as silicone. The wall **36** of the passageway **26** and the port 21 **b** are preferably formed from the same material.

The flange **28** may have any profile when viewed along the **A-A'** axis towards the distal **24** end, but in general, it will at least partially correspond with the shape of the nose mask **10.** The profile refers to the outer shape, or the profile of the opening **82,** or both. By correspond, it is meant that they essentially overlap, but some deviation is permitted, for example, the profile of the flange **28** may differ in area compared with the profile of the nose mask **10** by 0%, ±10%, ±15%, ±20%, ±25 % or a value between any two of the aforementioned values.

While the profile of flange **28** may at least partially corresponds with that of the nose mask **10** according to one embodiment, an independent description of the profile of the flange **28** now follows. The flange **28** is dimensioned to fit around the nose of the subject. It preferably extends from the nose around at least part of the cheeks. The flange **28** is disposed with an opening **82** for the nose that is preferably triangular in shape, but may alternatively be round, square, oblong or any other suitable shape. It may have the shape substantially of an isosceles trapezium. The shape may be largely determined by the shape of the nose. As will be appreciated, the flange **28** may be dimensioned so as not to extend over the eyes, mouth or ears. The flange **28** is devoid of apertures for the eyes, mouth or ears. However, an edge of the flange **28** may extend partially around, but not over, the eyes, mouth or ears.

The flange **28** may have a substantially longitudinal shape. With reference to **FIG. 8****,** the flange **28** has a longitudinal (w) direction, and perpendicular thereto, a lateral direction (h). The longitudinal (w) direction is aligned with the base of the nose (*i.e*. parallel to the eyes). The length in the lateral direction may be greater than that in the longitudinal direction. The opening **82** is preferably centrally located along the longitudinal length of the flange **28.** The opening **82** may also be centrally located with respect to the lateral height (h). The flange **28** thus comprises two flaps which extend longitudinally from and flank the opening **82,** and two flaps which extend laterally from and flank the opening **82.**

It is within the scope of the invention that the outer shape of the flange **28** is identical to that of the nose mask **10,** or corresponds at least partially to that of the nose mask **10.** The outer shape of the flange **28** may at least partially extend beyond the profile of the nose mask **10** or *vice versa.*

The dimension of the longitudinal width (WFO) of the flange **28** may be defined as the surface distance between the extremities of the opposing longitudinal edges of flange **28.** The distance is measured in the longitudinal (w) direction. The dimensions of the longitudinal width (WFO) will depend on the profile of the nose mask **10,** and may differ by 0%, ±10%, ±15%, ±20%, ±25 % or more.

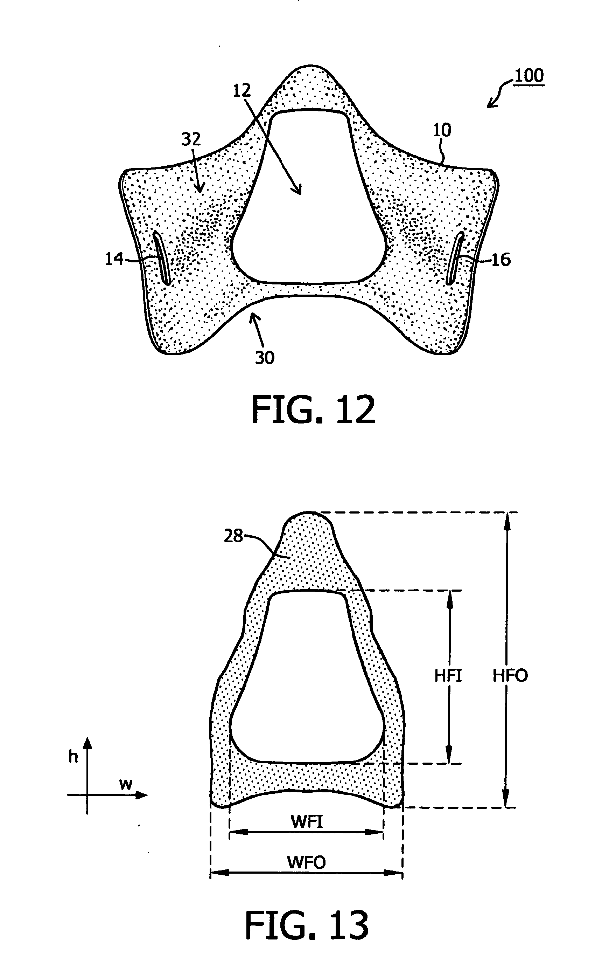

The dimension of the lateral height (HFO, **FIG. 13****)** of the flange **28** may be defined as the minimum surface distance between the extremities of the opposing lateral edges of the mask. The distance is measured along the lateral (h) direction. The dimensions of the lateral height (HFO) will depend on the profile of the nose mask **10,** and may differ by 0%, ±10%, ±15%, ±20%, ±25 % or more.

The dimension of the longitudinal width (WFO, **FIG. 13**) is preferably smaller than that of the lateral height (HFO) of the flange **28.** Preferably the dimension of the longitudinal width (WFO) is 1.5, 2, 2.5, 3, 4, 5, or 6 times smaller than that of the lateral height (HFO), or a value in the range between any two of the aforementioned values, preferably between 1.5 and 3 times.

The opening **82** for the nose is dimensioned such that the nose substantially or entirely passes there through. It is of sufficient size that at least the nasal tip passes entirely through the opening **82.** Preferably, it is of sufficient size that the nose passes entirely through the opening **82.** The opening **82** may be triangular, round, oblong, square of any other suitable shape. It may have the shape substantially of an isosceles trapezium. The shape will be largely determined by the shape of the nose. Preferably, the opening **82** is substantially isosceles trapezium in shape, the isosceles trapezium having a base that corresponds to the nostril region of the nose. The isosceles trapezium also has an upper part that corresponds to the nasal bone. The orientation of the isosceles trapezium is such that the base lies in the longitudinal direction (w) of the flange **28;** the triangle base does not lie in the lateral direction (h) of the flange **28.** Preferably the dimension of the base of the isosceles trapezium (WFI) is 1 cm, 2 cm, 3 m or 4 cm in length. Preferably the perpendicular height (HFI) of the isosceles trapezium relative to the base is 3cm, 4cm, or 5cm in length. The profile of the opening **82** of the flange **28** may be smaller in area compared with the profile of the aperture **12** of the nose mask **10,** by 0 %, 2 %, 4 %, 6 %, 8 %, 10 %, 15 %, 20 % or more.

The thickness of the flange **28** will generally be determined by the material used for construction. However, as a general guidance, the minimum thickness of the flange **28** will be equal to or less than 0.5 mm, 1 mm, 2 mm, 3 mm, 4 mm, or a value in the range between any two of the aforementioned values.

The length of the passageway **26** (from the flange **28** to the inlet port **21b**) is sufficient to receive the length of the nose, from the cheek to tip. As a general guidance, it may be 1 cm, 2 cm, 3 cm, 4 cm, 5 cm, 6 cm, 7 cm or longer, or a value in the range between any two of the aforementioned values.

According to another embodiment of the invention, the tubular fitting is identical to those described above and in **FIGs. 9A to 9E** above, except the inlet port **21, 21c** is not circular (21b) but is essentially triangular or has an essentially isosceles trapezium shape as shown in **FIGs. 10A** to **10E****.** The description above applies directly to the embodiments shown in **FIGs. 10A** to **10E** except for the non-circular port.

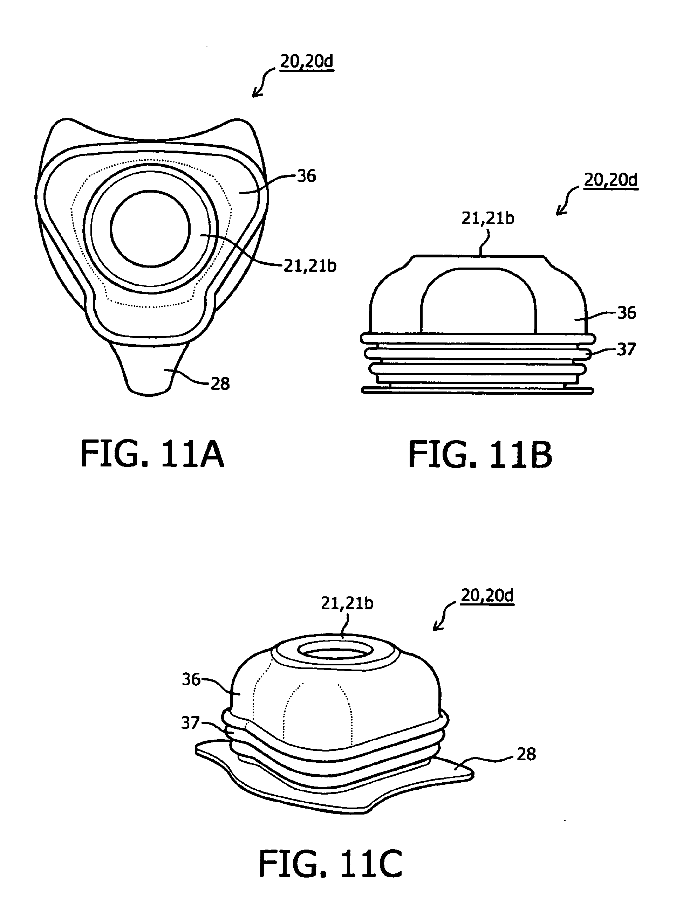

According to another embodiment of the invention, the tubular fitting 20, 20d is identical to those described above, and in **FIGs. 9A to 9E** and **FIGs. 10A** to **10E****,** except the wall of the passageway **36** is provided with a bellowed sub-region 37 as shown, for example, in **FIGs. 11A** to **11C****.** A bellowed region **37** is understood in the art, particularly in the application of bellowed tubing, and provides limited flexibility along the **A-A'** axis. The bellowed region **37** allows the coupling **20d** to bend and flex, thereby buffering tension applied to the coupling **20, 20d** by way of the attached gas tube. The bellows may be in abutting alignment with the flange, as shown in **FIGs. 11A** to **11C****.** The bellowed region may be provided along 10 %, 20 %, 30 %, 40 %, 50 %, 60 % of the length of the passageway **26** or a value between any two of the aforemented values. The description above applies directly to the embodiments shown in **FIGs. 11A** to **11C** except for the bellowed region.

According to another embodiment, the tubular fitting **20** is a triangular tube sealed over the nose aperture **12,** *i.e.* sealed to the mask **10** exterior surface **30** using any suitable technique, such as employing adhesive, welding methods. The tube is permanently sealed over the nose aperture **12.** The triangular tube is preferably made from a rubberized material, silicone, and is transparent.

According to another embodiment of the invention **(****FIGs. 13 to 15**), the coupling is comprised in an adaptor **45** comprising a pair of adjustable outlet nozzles **46, 46'** for outlet of gas in fluid connection with an inlet port **48** for coupling to a fitting for providing gas (*e.g.* air). The outlet nozzles **46, 46'** are positioned on the mask for entry into the nostrils. Each nozzle body is essentially parallel to the central lateral axis **(A-A').** The adapter **45** may be attached to the mask **10** exterior surface **30** using any suitable technique, such as employing adhesive, or welding methods. As shown in **FIGs. 13** to **15****,** the nozzles **46, 46'** are arranged in alignment with the base of the triangular aperture **12** which alignment orients the nozzles **46, 46'** for insertion into the nose after molding. An adapter-type coupling **45** having the above described configuration is known in the art, for instance, as a nasal pillow. Examples of manufacturers of nasal pillows include ADAM and Resmed. Typically, they have a substantially polypropylene or polycarbonate body.

According to another embodiment of the invention **(****FIGs. 16 to 18**), the coupling is comprised in a tube clip **40** configured to couple securely to a fluid delivery tube, such as a catheter, to the nose mask and align it with the entrance to a nostril. In **FIGs. 16** **and** **17****,** the tube clip **40** is visible and is positioned below the base of the triangular nose aperture **12.** The tube clip **40** may be any suitable for securing a catheter. The number of tube clips may be 1, 2, 3, 4, 5, 6 or more. Each tube clip may be adapted to secure 1, 2, 3, 4, 5, 6 or more tubes. The tube clip is preferably formed from a non-thermoplastic material such as polycarbonate or polypropylene.

## Claims

1. A wearable medical support (100) for delivery of fluids to the nose of a subject comprising:
- a heat moldable nose mask (10), having a longitudinal direction, formed from a sheet of thermoplastic material configured for extending and for individual molding across at least part of the cheek bones of the subject, comprising:
- a fixture (14, 16) for a strap at each opposing longitudinal end of the mask (10),
and
- a coupling (20, 20a-c, 40, 45) for attachment of one or more tubes for delivery of the fluid to the nose,
**characterized in that** it comprises a nose aperture (12) dimensioned to fit the nose of the subject and the coupling is dismountably fixed to the mask (10).

2. Support according to claim 1, where the coupling is comprised in a hollow tubular fitting (20a to c) having a proximal (22) and distal (24) end,
- the proximal end (22) of the tubular fitting (20a to c) being provided with a flanged (28) opening (82) for receiving the nose,
- the distal end (24) being provided with an inlet port (21) for attachment to a tube for delivery of the fluid to the nose, and
- a passageway (26) connecting the inlet port (21a to c) to the flanged opening (82).

3. Support according to claim 2, wherein the flange (28) is provided on a skin-facing side (32) of the nose mask (10), the inlet port (21) is provided on an exterior side (30) of the nose mask (10), and the passageway (26) is disposed through the nose aperture (12), so mounting the mask (10) over the passageway (26), and in abutting alignment with the proximal (22) side of the flange (28).

4. Support according to claim 2 or 3, wherein the inlet port (21b) and passageway (26) are configured to pass slidably through the nose aperture (12).

5. Support according to any of claims 2 to 4, wherein the passageway (26) is at least partially bellowed.

6. Support (100) according to any of claims 1 to 5, wherein the nose aperture (12) is essentially triangular or isosceles trapezoidal, the base of the triangle or isosceles trapezium oriented in the longitudinal direction of the nose mask (10).

7. Support (100), according to any of claims 1 to 6 wherein the longitudinal width (W) of the mask (10) is between 16 cm and 22 cm.

8. Support (100), according to any of claims 1 to 7, wherein the longitudinal width (W) of the mask (10) is larger than that of the lateral height (H) of the mask, preferably between 1.5 and 3 times larger.

9. Support (100), according to any of claims 1 to 8, wherein the nose mask (10) comprises a thermoplastic composition containing polycaprolactone and polyurethane.

10. Support (100), according to any of claims 1 to 9, wherein the nose mask (10) comprises is disposed with a plurality of aeration apertures.

11. Support (100), according to claim 10, wherein the aeration apertures are provided at a density of between 0.5 and 5 apertures per cm².

12. Support (100), according to any of claims 9 to 11, wherein the sheet of thermoplastic material comprises a core layer, and wherein the core layer (60) comprises 20 % to 40 %, polyurethane, and 60 % to 80 % (w/w) polycaprolactone.

13. Support (100), according to any of claims 2 to 12, wherein the flange (28) is formed from silicone.

14. Support (100), according to any of claims 1 to 13, wherein the overall thickness of the thermoplastic sheet is between 1.5 and 2 mm.

15. A coupling dismountably fixed to a heat moldable mask for attachment of one or more tubes for delivery of fluids to the nose, said coupling is provided with a flange (28) having a profile corresponding at least partially to that of the mask (10), the mask is having a nose aperture (12) dimensioned to fit the nose of the wearer.

## Patentansprüche

1. Ein tragbares medizinisches Unterstützungsgerät (100) für die Zulieferung von Flüssigkeiten in die Nase eines Patienten, umfassend:
- Eine heißformbare Nasenmaske (10), mit einer Längsrichtung, aus einer Folie aus thermoplastischem Material geformt, zum Ausbreiten und individuelles Formen über zumindest eines Teils der Backenknochen des Subjekts konfiguriert, umfassend:
- einer Befestigung (14, 16) für ein Band an jedem gegenüberliegenden Längsende der Maske (10),
und
- einer Kupplung (20, 20a-c, 40, 45) zum Anbringen von einer oder mehreren Röhren zur Abgabe der Flüssigkeit an die Nase,
**dadurch gekennzeichnet, dass** sie eine Nasenöffnung (12) beinhaltet, welche dimensioniert ist, um sich der Nase des Patienten anzupassen und dass die Kupplung an der Maske (10) abnehmbar befestigt ist.

2. Ein Unterstützungsgerät nach Anspruch 1, wobei die Kupplung in einem rohrförmigen Fitting (20a bis c) mit einem proximalen (22) und einem distalen (24) Ende umfasst ist,
- das proximale Ende (22) des rohrförmigen Fittings (20a bis c) mit einer geflanschten (28)Öffnung (82) zur Aufnahme der Nase vorgesehen,
- das distale Ende (24) mit einer Einlassöffnung (21) zur Befestigung an einer Röhre für die Flüssigkeitsabgabe an die Nase vorgesehen, und
- einen Durchgang (26), welcher die Einlassöffnung (21a bis c) mit der geflanschten Öffnung (82) verbindet.

3. Ein Unterstützungsgerät nach Anspruch 2, wobei der Flansch (28) auf einer der Haut zugewandten Seite (32) der Nasenmaske (10) versehen ist, die Einlassöffnung (21) auf einer Außenseite (30) der Nasenmaske (10) versehen ist und der Durchgang (26) durch die Nasenöffnung (12) aufgestellt wird, und in dieser Weise die Maske (10) über den Durchgang (26), und in angrenzender Ausrichtung mit der proximalen (22) Seite des Flansches (28) befestigt wird.

4. Ein Unterstützungsgerät nach einem der Ansprüche 2 oder 3, wobei die Einlassöffnung (21b) und der Durchgang (26) konfiguriert sind, um durch die Nasenöffnung (12) durchzugleiten.

5. Ein Unterstützungsgerät nach einem der Ansprüche 2 bis 4, wobei der Durchgang (26) zumindest teilweise gefaltet ist.

6. Ein Unterstützungsgerät (100) nach einem der Ansprüche 1 bis 5, wobei die Nasenöffnung (12) im Wesentlichen dreieckig oder gleichschenklig trapezförmig ist, wobei die Basis des Dreiecks oder des gleichschenkligen Trapezes in der Längsrichtung der Nasenmaske (10) ausgerichtet ist.

7. Ein Unterstützungsgerät (100), nach einem der Ansprüche 1 bis 6, wobei die longitudinale Weite (W) der Maske (10) zwischen 16 cm und 22 cm beträgt.

8. Ein Unterstützungsgerät (100), nach einem der Ansprüche 1 bis 7, wobei die longitudinale Weite (W) der Maske (10) größer ist, als diejenige der seitlichen Höhe (H) der Maske, vorzugsweise zwischen 1,5 und 3 mal größer.

9. Ein Unterstützungsgerät (100), nach einem der Ansprüche 1 bis 8, wobei die Nasenmaske (10) eine thermoplastische Zusammensetzung aus Polycaprolacton und Polyurethan umfasst.

10. Ein Unterstützungsgerät (100), nach einem der Ansprüche 1 bis 9, wobei die Nasenmaske (10) mit einer Vielzahl von Belüftungsöffnungen angeordnet ist.

11. Ein Unterstützungsgerät (100), nach Anspruch 10, wobei die Belüftungsöffnungen in einer Dichte von zwischen 0,5 und 5 Öffnungen pro cm2 vorgesehen sind.

12. Ein Unterstützungsgerät (100), nach einem der Ansprüche 9 bis 11, wobei die Folie aus thermoplastischem Material eine Kernschicht umfasst, und wobei die Kernschicht (60) zu 20% bis 40% Polyurethan und zu 60% bis 80% (w/w) Polycaprolacton umfasst.

13. Ein Unterstützungsgerät (100), nach einem der Ansprüche 2 bis 12, wobei der Flansch (28) aus Silikon gebildet ist.

14. Ein Unterstützungsgerät (100), nach einem der Ansprüche 1 bis 13, wobei die Gesamtstärke der thermoplastischen Folie zwischen 1,5 und 2 mm liegt.

15. Eine Kupplung, abnehmbar an eine Heißformbare Maske zum Anbringen einer oder mehrerer Röhren zur Abgabe von Flüssigkeiten an der Nase befestigt, wobei die Kupplung mit einem Flansch (28), dessen Profil mindestens teilweise dem der Maske (10) entspricht, vorgesehen ist, die Maske mit einer Nasenöffnung (12) versehen, welche dimensioniert ist, um sich der Nase des Trägers anzupassen.

## Revendications

1. Support médical portable (100) pour la distribution de fluides au nez d'un sujet, comprenant :
- un masque nasal thermomoulable (10), ayant une direction longitudinale, formé à partir d'une feuille de matériau thermoplastique configurée pour s'étendre et pour un moulage individuel sur au moins une partie des pommettes du sujet, comprenant :
- un dispositif de fixation (14, 16) pour une sangle au niveau de chaque extrémité longitudinale opposée du masque (10),
et
- un accouplement (20, 20a-c, 40, 45) pour le raccordement d'un ou de plusieurs tubes pour la distribution du fluide au nez,
**caractérisé en ce qu'**il comprend une ouverture pour le nez (12) dimensionnée pour s'adapter au nez du sujet et l'accouplement est fixé de manière démontable au masque (10).

2. Support selon la revendication 1, dans lequel l'accouplement est constitué d'un raccord tubulaire creux (20a à c) ayant une extrémité proximale (22) et une extrémité distale (24),
- l'extrémité proximale (22) du raccord tubulaire (20a à c) étant pourvue d'une ouverture (82) à bride (28) pour recevoir le nez,
- l'extrémité distale (24) étant pourvue d'un orifice d'entrée (21) pour le raccordement à un tube pour la distribution du fluide au nez, et
- un passage (26) reliant l'orifice d'entrée (21a à c) à l'ouverture à bride (82).

3. Support selon la revendication 2, dans lequel la bride (28) est située sur un côté face à la peau (32) du masque nasal (10), l'orifice d'entrée (21) est situé sur un côté extérieur (30) du masque nasal (10), et le passage (26) est disposé à travers l'ouverture pour le nez (12), montant ainsi le masque (10) par-dessus le passage (26) et en alignement par butée avec le côté proximal (22) de la bride (28).

4. Support selon la revendication 2 ou 3, dans lequel l'orifice d'entrée (21b) et le passage (26) sont configurés pour passer de façon coulissante à travers l'ouverture pour le nez (12).

5. Support selon l'une quelconque des revendications 2 à 4, dans lequel le passage (26) est au moins partiellement à soufflet.

6. Support (100) selon l'une quelconque des revendications 1 à 5, dans lequel l'ouverture pour le nez (12) est essentiellement triangulaire ou trapézoïdale isocèle, la base du triangle ou du trapèze isocèle étant orientée dans la direction longitudinale du masque nasal (10).

7. Support (100), selon l'une quelconque des revendications 1 à 6, dans lequel la largeur longitudinale (W) du masque (10) est comprise entre 16 cm et 22 cm.

8. Support (100), selon l'une quelconque des revendications 1 à 7, dans lequel la largeur longitudinale (W) du masque (10) est plus grande que celle de la hauteur latérale (H) du masque, de préférence entre 1,5 et 3 fois plus grande.

9. Support (100), selon l'une quelconque des revendications 1 à 8, dans lequel le masque nasal (10) comprend une composition thermoplastique contenant du polycaprolactone et du polyuréthane.

10. Support (100), selon l'une quelconque des revendications 1 à 9, dans lequel le masque nasal (10) comprend une pluralité d'ouvertures d'aération.

11. Support (100), selon la revendication 10, dans lequel les ouvertures d'aération sont prévues à une densité comprise entre 0,5 et 5 ouvertures par cm².

12. Support (100), selon l'une quelconque des revendications 9 à 11, dans lequel la feuille de matériau thermoplastique comprend une couche de base, et dans lequel la couche de base (60) comprend 20 à 40 % de polyuréthane et 60 à 80 % (M/M) de polycaprolactone.

13. Support (100), selon l'une quelconque des revendications 2 à 12, dans lequel la bride (28) est formée à partir de silicone.

14. Support (100), selon l'une quelconque des revendications 1 à 13, dans lequel l'épaisseur totale de la feuille thermoplastique est comprise entre 1,5 et 2 mm.

15. Accouplement fixé de manière démontable à un masque thermomoulable pour le raccordement d'un ou de plusieurs tubes pour la distribution de fluides au nez, ledit accouplement étant pourvu d'une bride (28) ayant un profil correspondant au moins en partie à celui du masque (10), le masque possédant une ouverture pour le nez (12) dimensionnée pour s'adapter au nez de l'utilisateur.
